# EUROPEAN PATENT APPLICATION

(11) **EP 1 738 796 A2**
(43) Date of publication of application: **03.01.2007**
(21) Application number: 05254226.3
(22) Date of filing: 06.07.2005
(51) Int. Cl.: A61N 2/06, B25H 3/00

(54) **Tool belt**

(30) Priority: 28.06.2005 US 169318
(71) Applicant: Contractor Pro, LLC, Sandy UT 84092 (US)
(72) Inventor: Newman, Kirk B., Sandy, Utah 94092 (US); Liu, Alex Yu-Feng, Glendora, California 91706 (US)
(74) Representative: Thomson, Paul Anthony

(57) **Abstract**

A tool belt (10) for fastening about a wearer's torso comprises a support belt (12) having a first end (14) and a second end (16) each having attachment means (15,17) for securing the belt about the wearer's torso. A plurality of individual cushion pads (18) is attached to the support belt on a proximal surface (20) of the support belt. At least one of a plurality of magnetic elements (22) is associated with one of the individual cushion pads. The individual cushion pad has an indentation (24) formed in a proximal surface (26) thereof, shaped to correspond to a shape of the at least one magnetic element, and the at least one magnetic element is at least partially disposed within the indentation in the cushion pad to restrict movement of the magnetic element relative to the cushion pad while the belt is in use about the wearer's torso.

## Description

### BACKGROUND:

### Field of the Invention:

The present invention relates generally to belts for storing and carrying tools. More particularly, the present invention relates to tool belts that provide a therapeutic benefit to the wearer and that are easily handled by a wearer.

### Related Art:

Tool belts have been used for some time to carry or store a variety of tools, fasteners, equipment, etc., about a wearer's waist or torso. Such belts typically include pouches, hooks, clips and similar structure on or in which tools and equipment are carried by the belt while the belt is secured about the individual's waist. In this manner, as the individual travels from one working location to another, he or she can ensure that the tools and equipment necessary for a particular job are available for use in any particular location.

While tool belts have become much needed accessories for individuals in a variety of trades, they continue to suffer from a number of problems. For example, storage of tools and equipment on a tool belt necessarily adds, sometimes significantly, to the weight of the belt. As the weight of a loaded tool belt increases, detrimental stress applied to the wearer's back and body increases. This problem is often exacerbated by the fact that many tool belts are not "loaded" uniformly, e.g., they are often heavier on one side or another, increasing the detrimental stress or strain applied to a wearer's back. Increased stress or strain on a wearer's back can result not only in discomfort, but can lead to injury that can result in loss of productivity and morale, and may require expensive medical procedures.

In addition, the types of tools and equipment often stored or carried on a tool belt have disparate shapes, lengths and weights. While the tool belt is generally capable of adequately storing such tools when the belt is strapped about a waist of the individual, many of the pouches, hooks or clips are designed to properly hold tools and other devices only when the belt is in an upright orientation. This design not only allows the belt to be used to store a variety of odd-shaped tools and equipment, but also allows relatively easy insertion and withdrawal of the tools from the belt by the individual.

As a result, in the event the belt is tipped, or not otherwise held in an upright orientation, the tools and equipment can easily fall out of the pouches, hooks or other storage structure. Most wearers of such tool belts become accustomed to this and compensate for this fact by maintaining a generally upright posture when wearing the belt. However, when the individual removes the tool belt it can be very difficult to retain all portions of the belt in an upright configuration white holding or transporting the belt by hand, and carrying the belt by hand can result in tools or equipment falling from the belt.

### SUMMARY OF THE INVENTION

It has been recognized that it would be advantageous to develop a tool belt that provides a therapeutic benefit to a wearer of the tool belt during use of the tool belt. In addition, it has been recognized that it would be advantageous to develop a tool belt that can be readily manipulated or handled while not in use without dislodging tools or equipment from the belt.

The present invention provides a tool belt for fastening about a wearer's torso, including a support belt configured to be disposed about the wearer's torso and having a first end and a second end. The first and the second end can each have attachment means for securing the belt about the wearer's torso. A plurality of individual cushion pads can be attached to the support belt on a proximal surface of the support belt. A plurality of magnetic elements can also be provided, at least one of which is associated with one of the individual cushion pads. The individual cushion pad can have an indentation formed in a proximal surface thereof, the indentation being shaped to correspond to a shape of the at least one magnetic element. The at least one magnetic element can be at least partially disposed within the indentation in the cushion pad to restrict movement of the magnetic element relative to the cushion pad while the belt is in use about the wearer's torso.

In accordance with another aspect of the invention, a tool belt for fastening about a wearer's torso is provided, including a support belt having a first end and a second end. The first end and the second end can each have attachment means for securing the belt about the wearer's torso. A plurality of individual cushion pads can be attached to the support belt on a proximal surface of the support belt. A plurality of magnetic elements can also be provided, each being coupled to a proximal surface of one of the individual cushion pads. Each of the individual cushion pads can have at least two configurations: i) a first, relaxed configuration in which the magnetic element associated with the individual cushion pad protrudes partially above the proximal surface of the cushion pad; and ii) a second, compressed configuration in which the magnetic element is compressed into the individual cushion pad in substantially a same plane as the proximal surface of the cushion pad.

In accordance with another aspect of the invention, a tool belt for fastening about a wearer's torso is provided, including a support belt having a first end and a second end. The first end and the second end can each have attachment means for securing the belt about the wearer's torso. At least one handle can be attached to the tool belt, the handle being operable to receive a hand of the wearer to enable the wearer to support the tool belt with his or her hand.

Additional features and advantages of the invention will be apparent from the detailed description which follows, taken in conjunction with the accompanying drawings, which together illustrate, by way of example, features of the invention.

### BRIEF DESCRIPTION OF THE DRAWINGS

Additional features and advantages of the invention will be apparent from the detailed description which follows, taken in conjunction with the accompanying drawings, which together illustrate, by way of example, features of the invention, and wherein:
FIG. 1A is a front view showing a proximal side of a tool belt in accordance with an embodiment of the present invention;
FIG. 1B is a rear view showing a distal side of the tool belt of FIG. 1A;
FIG. 2 is a side view of a cushion pad section of the tool belt of FIG. 1A, taken along section A-A of FIG. 1A;
FIG. 3 is another view of the cushion pad of FIG. 2, shown in a compressed configuration;
FIG. 4A is a side view of another cushion pad section of the tool belt of FIG. 1A, taken along section A-A of FIG. 1A; and
FIG. 4B is an exploded view of cushion pad section of FIG. 4A.

Reference will now be made to the exemplary embodiments illustrated, and specific language will be used herein to describe the same. It will nevertheless be understood that no limitation of the scope of the invention is thereby intended.

### DETAILED DESCRIPTION

Reference will now be made to the exemplary embodiments illustrated in the drawings, and specific language will be used herein to describe the same. It will nevertheless be understood that no limitation of the scope of the invention is thereby intended. Alterations and further modifications of the inventive features illustrated herein, and additional applications of the principles of the inventions as illustrated herein, which would occur to one of ordinary skill in the relevant art and having possession of this disclosure, are to be considered within the scope of the invention.

In describing and/or claiming the present invention, the following terminology may be used:
As used herein, the term "proximal" is to be understood to refer to a location that is nearer to a wearer of a tool belt than to persons or things about the wearer. Similarly, the term "distal" is to be understood to refer to a location that is nearer persons or things about the individual than to the wearer of the tool belt. Thus, in the case where a tool belt is strapped about a person, the surface or "side" of the tool belt that contacts the wearer's back, sides and stomach is the proximal surface or side of the tool belt. In this example, the proximal side of the tool belt extends about and is exposed toward (or is in contact with) the wearer's torso.
As used herein, the terms "inseparably coupled" and "inseparably attached" are to be understood to refer to a condition in which two or more structures are stitched, bonded, melded, attached, or otherwise joined to one another in such a manner that they cannot be separated from one another without causing structural damage to at least one of the two or more structures. The term "inseparably coupled" does not apply, for example, to two or more materials that are merely held adjacent one another, or one within another, and are capable of being separated during normal usage of a device comprised of the materials.

As illustrated in the attached figures, in one exemplary embodiment, the present invention generally provides a tool belt 10 for fastening about a wearer's torso. The tool belt can generally be attached about the wearer's waist or torso in a similar manner as conventional tool belts are worn. Due to the similar nature of this aspect of the invention to conventional tool belts, no individual or wearer is illustrated in the figures, it being understood that one of ordinary skill in the art could readily adapt the present invention for use with individual wearers.

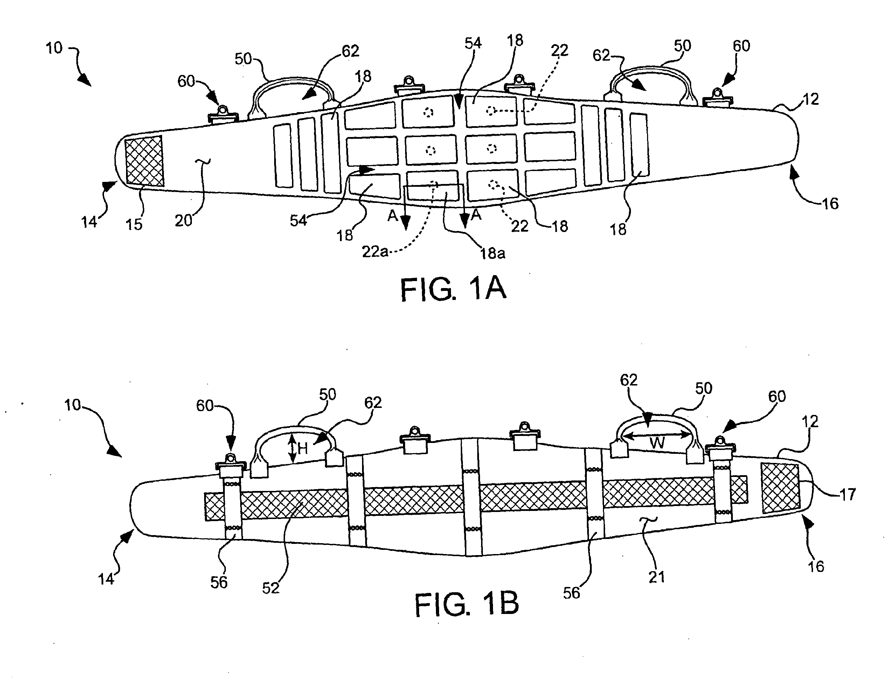

As shown in detail in FIGs. 1A-3, the tool belt 10 can generally include a support belt 12 that can be configured to be disposed about the wearer's torso and can have a first end 14 and a second end 16. The first and second ends can each have attachment means 15 and 17, respectively, associated therewith for securing the belt about the wearer's torso. The attachment means can be of a variety known to those of ordinary skill in the art, including buckles, straps, hook-and-loop fasteners, snaps, buttons, etc.

A plurality of individual cushion pads 18 can be attached to the support belt 12 on a proximal surface 20 of the support belt. The pads can provide a cushioned interface between the wearer and the belt to provide a more comfortable working environment for the wearer. The cushion pads can generally be conformable and compressible and can be formed from a variety of materials, including open-cell foam and similar materials. A plurality of magnetic elements 22 can be provided, at least one of which can be associated with one of the individual cushion pads. In the example shown in FIGs. 1A, 2 and 3, magnetic element 22a is associated with cushion pad 18a.

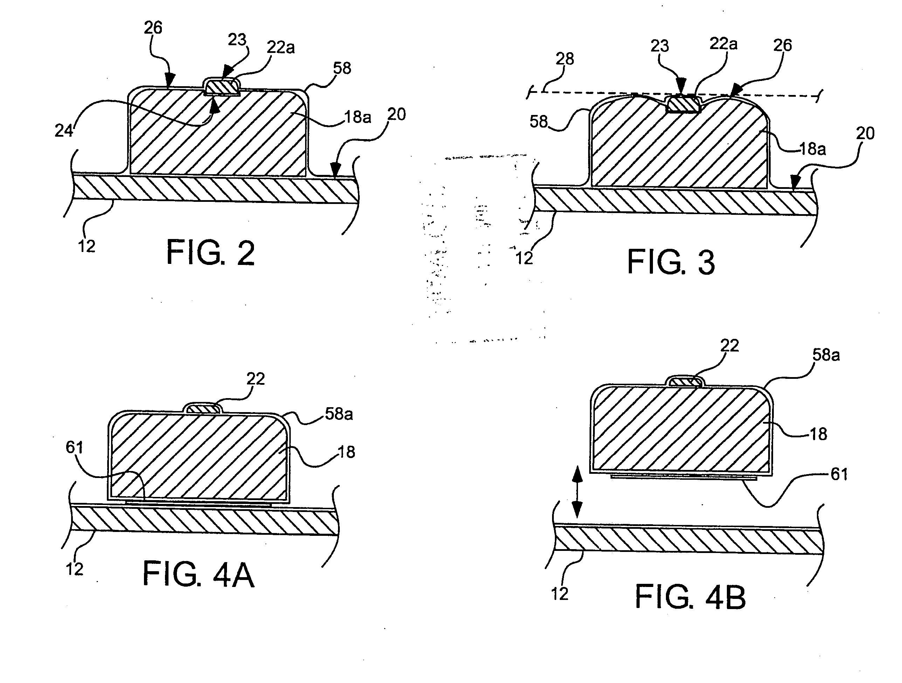

As best appreciated from viewing FIG. 2, the individual cushion pad 18a can have an indentation 24 formed in a proximal surface 26 thereof. The indentation can be shaped to correspond to a shape of the magnetic element 22a. The at least one magnetic element can be at least partially disposed within the indentation in the cushion pad to restrict lateral movement of the magnetic element relative to the cushion pad while the tool belt is in use about the wearer's torso. While not so required, in one aspect of the invention, the magnetic element is bonded or attached within the indentation to aid in retaining the magnetic element within the indentation.

In the embodiments illustrated in the figures, the magnetic elements 22 and the indentations 24 are generally circular in shape. However, the magnetic elements and the indentations can be formed in a variety of shapes, including square, rectangular, oval, etc., as would occur to one skilled in the relevant art. Also, while the size of the magnetic elements is shown as generally much smaller than the cushion pads, it is to be understood that the magnetic elements can be larger or smaller, relative to the cushion pad, depending upon the particular application in which the magnetic elements and cushion pads are to be used.

It will thus be appreciated that the present invention provides a tool belt that includes both cushion pads 18 that provide a conformable, comfortable interface between the tool belt and the wearer of the tool belt, and magnetic elements 22 that can provide a therapeutic benefit to the wearer. The benefits of magnet therapy in relieving pain and stiffness have been studied for some time. In general, magnetic therapy involves the placement of magnetic devices on or near the body to relieve pain and facilitate healing or sore muscles, joints, etc.

While the precise mechanism through which magnetic therapy operates has not been clearly proven, advocates of magnetic therapy claim that such therapy has a positive effect on the body, particularly in relieving pain. In general, it is believed that the magnetic fields produced by magnets (or by devices that generate electromagnetic current) can penetrate the human body and affect the functioning of individual cells and improve the working of the nervous system and various organs.

The present invention thus provides a tool belt having the beneficial aspects of magnetic therapy coupled, in some embodiments, with the therapeutic benefit of compressible support cushion pads. The magnetic elements 22 used in the present invention can be of a variety of types, and in one embodiment are permanent magnets that generate a magnetic field ranging from 250 to 750 gauss. In one embodiment of the invention, the magnetic elements are permanent magnets that generate a magnetic field of about 500 gauss.

In the case where the magnetic elements used are moderately to highly directional, the magnetic elements can be disposed on or coupled in or to the cushion pads such that the magnetic field generated by the magnetic elements is directed away from the proximal surface of the belt toward the wearer. While permanent magnets are used in some embodiments of the invention, the magnetic elements can also be temporary magnets and/or electromagnets, as would occur to one having ordinary skill in the art. In those aspects of the invention where electromagnets are used, a suitable power supply (not shown) can be provided on or near the tool belt to provide power to the electromagnets.

As shown in FIGs. 2 and 3, in one aspect of the invention the magnetic element 22a can protrude at least partially above the proximal surface 26 of the cushion pad 18a. In this manner, as the wearer dons the tool belt, the magnetic element contacts the wearer first (or, in most cases, contacts the clothing of the wearer first). As the belt is cinched about the wearer, the magnetic element is compressed into the cushion pad until a proximal surface 23 of the magnetic element and the proximal surface of the cushion pad are aligned in substantially the same plane, as shown schematically by plane 28 in FIG. 3. Thus, in this aspect of the invention, the individual cushion pad includes: i) a first, relaxed configuration in which the at least one magnetic element protrudes partially above the proximal surface of the cushion pad; and ii) a second, compressed configuration in which the magnetic element is compressed into the individual cushion such that the proximal surface of the magnetic element is substantially level with the proximal surface of the cushion pad.

The individual cushion pad 18 can thus circumscribe the at least one magnetic element 22 to form a cushioned area of contact with a back of the wearer about the at least one magnetic element. In this manner, the portion of the support belt 12 contacting the wearer (or the wearer's clothes) includes a magnetic contact interface surrounded by a cushion pad interface. This configuration can maximize the therapeutic benefit provided by the tool belt.

The number of cushion pads and magnetic elements provided on the belt can vary according to the particular application of the tool belt. For example, in the embodiment illustrated in FIG. 1A, eighteen cushion pads 18 are provided with only the innermost six cushion pads having a magnetic element 22 associated therewith. Thus, in this aspect of the invention, a plurality of cushion pads are provided but only a portion of that plurality includes a magnetic element. In addition, each of the cushion pads with which a magnetic element is associated can include only a single magnetic element, or can include multiple magnetic elements, depending upon the particular application in which the tool belt is to be used.

The magnetic elements 22 can be disposed on the proximal 26 surface of the cushion pads 18 in a variety of locations, and in one embodiment the magnetic element is substantially centered on the proximal surface of the cushion pad, in both a horizontal and a vertical aspect. This aspect of the invention can maximize the therapeutic benefit of both the magnetic interface and the cushion interface between the tool belt and the wearer's torso by providing ample cushion surface about all sides of the magnetic element.

In the embodiment illustrated in FIG. 1A, the plurality of individual cushion pads 18 are each separated by an air space 54. The air space can be configured to allow at least a degree of compression of each pad independently of an adjacent pad. This aspect of the invention allows the support belt 12 to conform to the possibly unique curvature of a wearer's torso by allowing variable degrees of compression of individual cushion pads. In addition, the air space between adjacent cushion pads can allow air to circulate between the cushion pads to provide ventilation to aid in maintaining a cool and dry environment between the support belt and the wearer's torso.

As shown in FIG. 2, the support belt 12 can include a substantially continuous, conformable cover 58 that can be applied over the cushion pads and the magnetic elements to integrate the pads and magnetic elements into a single unit. In the aspect of the invention illustrated in FIGs. 4A and 4B, each cushion pad and magnetic element can be covered by an individual cover 58a. This aspect of the invention can be advantageous in embodiments of the invention in which the cushion pads are individually removable from the support belt 12 to allow a wearer to customize the cushion pad arrangement to tailor the belt for his or her own preferences. The cushion pad 18 of FiGs. 4A and 4B can be attached by attachment means 61, such as a hook-and-loop attachment system component that can securely but removably attach the individual cushion pads to the support belt. Thus, the pads can be relatively easily removed from the support belt and can be reattached in a different location.

The tool belt 10 can include a variety of hooks, pouches, straps, etc. (not shown in the figures) to allow tools and equipment to be stored on or in the tool belt, as would occur to one of ordinary skill in the art. In one aspect of the invention, a strip 52 of a portion of a hook-and-loop fastener system can be adhered to a distal surface 21 of the support belt 12 to aid in securing a variety of pouches, straps, etc. to the belt. In a typical scenario, a tool pouch (not shown) can be looped or extended about the belt and the strip 52 can engage a corresponding fastener material on the pouch to aid in securing the tool pouch from moving relative to the belt. The tool pouch can be of a variety of types known to those skilled in the art and can be configured to hold tools and fasteners such as nails, screws, etc.

As shown in FIG. 1B, the support belt 12 can include straps 56 that can allow an auxiliary belt (not shown) to be attached about the support belt to provide additional support to the support belt to secure the tool belt about a wearer's torso. Eyelets 60 can also be coupled to the support belt to allow suspenders (not shown) to be attached to the support belt to provide additional support to the tool belt.

Returning to FIGs. 1 and 2, a tool belt in accordance with another aspect of the invention can include support belt 12 that can be configured to be disposed about the wearer's torso. At least one handle 50 can be attached to the tool belt and can be operable to receive a hand (not shown) of the wearer to enable the wearer to support or carry the tool belt with his or her hand. This aspect of the invention can be advantageous in carrying the tool belt from one location to another, installing the tool belt about a waist, storing the tool belt in a vehicle, etc. As the handles 50 can be coupled directly to the tool belt, the weight of the tool belt can be supported securely by a hand of the wearer, and the wearer can carry the belt without dislodging tools or equipment from the tool belt.

In the aspect of the invention shown in FIGs. 1A and 1B, the belt 10 includes two handles 50. However, the belt can include a single or multiple handles, as a particular application may dictate. The placement of the handles relative to a length of the support belt 12 can also vary. For example, each of the handles 50 can be attached on opposing sides of the support belt and can be substantially centered lengthwise on each of two halves of the belt. In this manner, when both of the handles are grasped by a wearer, the tool belt as a whole is supported by the handles and the tool belt can be safely transported, stored, etc., without causing tools and equipment to fall from the tool belt. Thus, tools and equipment need not be "unloaded" from the tool belt prior to removing the belt from about a torso, or transporting or storing the tool belt.

The size, location and configuration of the handles 50 can vary, and in one embodiment the handles define a hand opening 62 between the handle and the belt. The hand opening can have a height H that is between about 1 inch and about 4 inches, and in one embodiment is about 1 ½ inches. The hand opening can have a width between about 4½ inches to about 6 inches. By restraining the shape of the hand opening defined by the handles, the hand opening can be sufficiently large to allow even a gloved hand to enter the hand opening, yet the handles do not extend above the belt to such an extent that the handles will interfere with the storage and retrieval of tools and equipment from the belt.

While not so required, the handles 50 shown in the exemplary embodiments of FIGs. 1A and 1B are inseparably attached to the support belt 12. In the embodiment shown, the handles are inseparably attached by stitching the handles to the support belt. In this manner, the handles form an integral part of the tool belt and are not susceptible to sliding or moving relative to the length of the tool belt, even in those cases where the tool belt is not loaded evenly.

This feature of the invention is advantageous in that tool belts are often not loaded evenly, and one side of the belt may be much heavier than another side. In cases where the handles are not fixed relative to the belt, heavier portions of the belt can tend to slide or slump away from a handle, potentially resulting in tools or equipment falling or becoming dislodged from the belt. The present invention addresses this problem by fixing the handles relative to the length of the belt to ensure that the full weight of the belt is supported by the handles and that the belt cannot slide relative to the handles.

It is to be understood that the above-referenced arrangements are only illustrative of the application for the principles of the present invention. Numerous modifications and alternative arrangements can be devised without departing from the spirit and scope of the present invention. While the present invention has been shown in the drawings and fully described above with particularity and detail in connection with what is presently deemed to be the most practical and preferred embodiment(s) of the invention, it will be apparent to those of ordinary skill in the art that numerous modifications can be made without departing from the principles and concepts of the invention as set forth herein.

## Claims

1. A tool belt for fastening about a wearer's torso, comprising:
a support belt having a first end and a second end, the first and second ends each having attachment means for securing the belt about the wearer's torso;
a plurality of individual cushion pads attached to the support belt on a proximal surface of the support belt;
a plurality of magnetic elements, at least one of which is associated with one of the individual cushion pads;
the individual cushion pad having an indentation formed in a proximal surface thereof, the indentation being shaped to correspond to a shape of the at least one magnetic element; and
the at least one magnetic element being at least partially disposed within the indentation in the cushion pad to restrict movement of the magnetic element relative to the cushion pad while the belt is in use about the wearer's torso.

2. The tool belt of claim 1, wherein the at least one magnetic element protrudes at least partially above the proximal surface of the cushion pad.

3. The tool belt of claim 1, wherein the individual cushion pad circumscribes the at least one magnetic element to form a cushioned area of contact with a back of the wearer about the at least one magnetic element.

4. The tool belt of claim 1, wherein the individual cushion pad includes:
a first, relaxed configuration in which the at least one magnetic element protrudes partially above the proximal surface of the cushion pad; and
a second, compressed configuration in which the magnetic element is compressed into the individual cushion such that a proximal surface of the magnetic element is substantially level with the proximal surface of the cushion pad.

5. The tool belt of claim 1, wherein only one magnetic element is associated with each cushion pad, the one magnetic element being substantially centered on the proximal surface of the cushion pad.

6. A tool belt for fastening about a wearer's torso, comprising:
a support belt having a first end and a second end, the first and second end each having attachment means for securing the belt about the wearer's torso; and
at least one handle, attached to the support belt, the handle being operable to receive a hand of the wearer to enable the wearer to support the tool belt with his or her hand.

7. The tool belt of claim 6, further comprising a second handle attached to the support belt, the second handle being operable to receive the hand of the wearer enable the wearer to support the tool belt from both handles with his or hand.

8. The tool belt of claim 7, wherein the handle and the second handle are attached on opposing sides of the support belt.

9. The tool belt of claim 6, wherein the handle defines a hand opening between the handle and the support belt, the hand opening having a height between about 1 inch and about 4 inches.

10. The tool belt of claim 6, wherein the handle is inseparably attached to the support belt.

11. The tool belt of claim 6, further comprising:
a plurality of individual cushion pads attached to the support belt on a proximal surface of the support belt;
a plurality of magnetic elements, each associated with one of the individual cushion pads;
the individual cushion pads each having an indentation formed in a proximal surface thereof, the indentations being shaped to correspond to a shape of the magnetic elements; and
the magnetic elements being at least partially disposed within the indentations in the cushion pad to restrict movement of the magnetic elements relative to the cushion pads while the belt is in use about the wearer's torso.
